(19) Europäisches Patentamt
European Patent Office
Office européen des brevets

(11) Veröffentlichungsnummer: **0 259 251 B1**

(12) # EUROPÄISCHE PATENTSCHRIFT

(45) Veröffentlichungstag der Patentschrift:
**27.02.91 Patentblatt 91/09**

(51) Int. Cl.[5]: **C07C 275/14,** C07D 295/12, C07D 521/00, D06P 1/66

(21) Anmeldenummer: **87810424.9**

(22) Anmeldetag: **27.07.87**

(54) **Kationische Umsetzungsprodukte aus basischen Carbamiden und Epihalogenhydrinen.**

(30) Priorität: **31.07.86 CH 3082/86**

(43) Veröffentlichungstag der Anmeldung:
**09.03.88 Patentblatt 88/10**

(45) Bekanntmachung des Hinweises auf die Patenterteilung:
**27.02.91 Patentblatt 91/09**

(84) Benannte Vertragsstaaten:
**BE CH DE ES FR GB IT LI**

(56) Entgegenhaltungen:
**GB-A- 2 126 579**

(56) Entgegenhaltungen:
**"CRC Handbook of Chemistry and Physics, Ausgabe 53, The Chemical Rubber Co., Ohio US, Seite C-441"; "s-Triazines and Derivatives, The Chemistry of Heterocyclic Compounds, E. M. Smolin und L. Rapoport, Interscience, NY US, 1967, Seiten 536-537"**

(73) Patentinhaber: **CIBA-GEIGY AG**
**Klybeckstrasse 141**
**CH-4002 Basel (CH)**

(72) Erfinder: **Töpfl, Rosemarie**
**Dorneckstrasse 68**
**CH-4143 Dornach (CH)**
Erfinder: **Binz, Jörg**
**Habshagstrasse 6/2**
**CH-4153 Reinach (CH)**

**EP 0 259 251 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Direktfarbstoffe lassen sich nicht oder nur mit ungenügender Farbausbeute im Pad-Batch-Verfahren auf Cellulosefasern applizieren. Werden dunkle Nuancen gefordert, so müssen diese Fasern in einem Ausziehverfahren gefärbt werden. Dies ist sehr aufwendig, da erhöhte Energie- und Chemikalienkosten anfallen. Ausserdem sind solche Färbungen, was den Echtheitsstandard betrifft, nicht allen Anforderungen gewachsen. Trotz Nachbehandlung mit konventionellen kationischen Nachbehandlungsmitteln sind solche Färbungen gegen die heute üblichen 60°C-Wäschen in Gegenwart von Natriumperborat nicht beständig, was ihre Einsatzmöglichkeit besonders auf dem Bekleidungssektor stark einschränkt.

Mit der Verwendung von Rekativfarbstoffen hoffte man das Problem der Nassechtheiten, vor allem der Waschechtheit, gelöst zu haben, weil diese Farbstoffe mit der Cellulose eine chemische Bindung eingehen. Es stellte sich jedoch heraus, dass die Farbstoff/Faserbindung gegen saure Hydrolyse nicht genügend beständig ist, was sich wiederum in einer Verschlechterung der Nassechtheiten und besonders in den Waschechtheiten ausdrückte. Der nicht mehr chemisch fixierte Farbstoff wird im Waschbad gelöst und kann daher z.B. ein anderes im gleichen Waschvorgang mitgewaschenes Textilgut anfärben. Ein weiterer Nachteil beim Färben mit Reaktivfarbstoffen ist der hohe Farbstoffverlust beim Auswaschen der Färbung, da nicht aller eingesetzter Farbstoff mit der Faser chemisch gebunden wird. Je nach Farbstofftyp können Auswaschverluste bis zu 60% festgestellt werden. Um eine einwandfreie Färbung zu erhalten benötigt man daher sehr aufwendige Wasch- und Spülvorgänge, um den nichtfixierten Farbstoff von der Faser zu entfernen.

Aus der GB-A-2 126 579 A sind polyquaternäre Ammoniumsalze bekannt, die beispielsweise durch Umsetzung von N,N-Bis-(dimethylaminoethyloder -propyl)-harnstoff mit 2 Mol Epichlorhydrin erhalten werden. Diese dort genannten Bischlorhydrine werden als Antimikrobika, Antistatika, Entmischungsmittel, Flockungsmittel, Korrosionsschutzmittel, Weichmacher verwendet.

Es wurden nun neue kationische Hilfsmittel auf Basis von Carbamiden aufgefunden, welche besonders die Nassechtheiten von Cellulosefärbungen, die mit substantiven Farbstoffen erzeugt werden, bedeutend verbessern und zudem eine Steigerung der Farbausbeute bewirken. Diese Produkte können sowohl in einer Vorbehandlung oder direkt beim Färben des Cellulosematerials eingesetzt werden.

Gegenstand vorliegender Erfindung sind daher quaternäre Ammoniumsalze der Formel

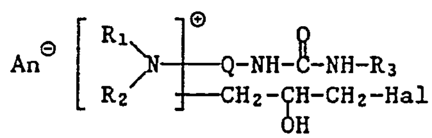 (1)

worin Q Alkylen mit 2 bis 6 Kohlenstoffatomen,

$R_1$ und $R_2$, unabhängig voneinander, je unsubstituiertes oder durch Halogen, Hydroxyl, Cyano oder Niederalkoxy substituiertes Niederalkyl, Cycloalkyl oder Aralkyl,

oder $R_1$ und $R_2$ zusammen mit dem sie verbindenden Stickstoffatom einen fünf- oder sechsgliedrigen heterocyclischen Rest, der gegebenenfalls ein weiteres Heteroatom enthält,

$R_3$ Wasserstoff, Niederalkyl oder Hydroxyniederalkyl,

Hal ein Halogenatom,

und $An^{\ominus}$ das Anion einer starken anorganischen oder organischen Säure bedeuten und die Halogenhydringruppe an ein quaterniertes Stickstoffatom gebunden ist.

Halogen bedeutet beispielsweise Fluor, Brom, Jod oder vorzugsweise Chlor.

Niederalkyl und Niederalkoxy stellen solche Gruppen oder Gruppenbestandteile dar, die 1 bis 5, insbesondere 1 bis 3 Kohlenstoffatome aufweisen, wie z.B. Methyl, Ethyl, n-Propyl, Isopropyl, n-Butyl, sek.-Butyl, tert.-Butyl oder Amyl oder Isoamyl bzw. Methoxy, Ethoxy, Isopropoxy, tert. Butoxy oder tert. Amyloxy.

Bei der Alkylengruppe Q handelt es sich beispielsweise um die

$-CH_2CH_2-$, $-CH_2CH_2CH_2-$, 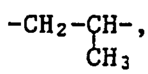 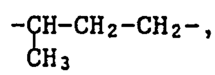 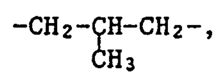

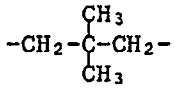

oder $-CH_2CH_2CH_2CH_2-$Gruppe

Vorzugsweise ist Q die Ethylen-, 2,2-Dimethylpropylen- oder vor allem die Propylengruppe $-CH_2CH_2CH_2-$.

Die Alkylreste in der Bedeutung von $R_1$ und $R_2$ können geradkettig oder verzweigt sein. Beispiele hierfür sind Methyl, Ethyl, n-Propyl, Isopropyl, n-Butyl, sek. Butyl, tert. Butyl, n-Amyl, Isoamyl oder tert. Amyl.

Sind die Alkylreste in $R_1$ und $R_2$ substituiert, so handelt es sich vor allem um Cyanoalkyl, Halogenalkyl, Hydroxyalkyl oder Alkoxyalkyl mit insgesamt vorzugsweise 2 bis 6 Kohlenstoffatomen, wie z.B. β-Cyanoethyl, β-Hydroxyethyl, γ-Hydroxypropyl, β-Methoxyethyl oder β-Ethoxyethyl. Als Hydroxyalkyl für $R_3$ ist besonders Hydroxymethyl (Methylol) zu nennen.

Beispiele für Cycloalkyl in der Bedeutung von $R_1$ und $R_2$ sind Cyclopentyl oder vorzugsweise Cyclohexyl. Die Cycloalkylreste können einen oder mehrere $C_1$-$C_4$-Alkylreste, vorzugsweise Methylgruppen, enthalten. Vorzugsweise weisen sie insgesamt 5 bis 10 Kohlenstoffatome auf.

Wenn $R_1$ und $R_2$ zusammen mit dem gemeinsamen Stickstoffatom einen heterocyclischen Rest darstellen, so kann dieser von einem ungesättigten oder gesättigten, fünf- oder sechsgliedrigen Stickstoffheterocyclus abgeleitet sein, der zusätzlich zum Stickstoffatom ein weiteres Heteraotom, wie Sauerstoff, Schwefel oder auch ein zweites Stickstoffatom enthalten kann.

$-NR_1R_2$ kann somit beispielsweise Pyrrolidino, Piperidino, Pipecolino, Morpholino, Thiomorpholino, Piperazino, Pyrazolino, Pyrazolidinyl oder Imidazolyl darstellen. Piperazino kann durch Niederalkyl oder Benzyl am zweiten Stickstoffatom substituiert sein.

Besonders bevorzugte heterocyclische Reste für $-NR_1R_2$ sind Pyrrolidino, Piperidino, Morpholino oder Imidazolyl.

$R_3$ bedeutet vorzugsweise Wasserstoff.

Die Halogenhydringruppe ist in erster Linie

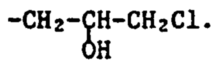

bildet in der Regel die quaternäre Ammoniumgruppe mit $-NR_1R_2$. Sie kann jedoch auch an ein zweites quaternisierbares Ringstickstoffatom in $-NR_1R_2$ gebunden sein.

Als Anionen $An^{\ominus}$ kommen sowohl Anionen anorganischer Säuren, wie z.B. das Chlorid-, Bromid-, Fluorid-, Jodid-, Sulfat- oder Phosphat-Ion als auch organischer Säuren, z.B. aromatischer oder aliphatischer Sulfonsäuren, wie z.B. Benzolsulfonat-, p-Toluolsulfonat-, Chlorbenzolsulfonat-, Methan- oder Ethansulfonat-Ion, ferner die Anionen niederer Carbonsäuren, wie z.B. Acetat-, Propionat- oder Oxalation.

$An^{\ominus}$ ist vor allem das Chlorid-, Bromid- oder Sulfation.

Praktisch wichtige kationische Verbindungen entsprechen der Formel

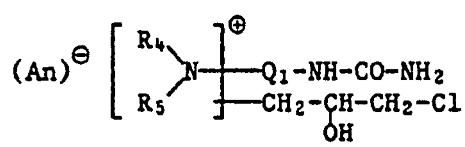

(2)

worin

$R_4$ und $R_5$ unabhängig voneinander, je Niederalkyl, besonders Methyl oder Ethyl, oder $-NR_3R_4$ Pyrrolidino, Piperidino, Morpholino oder Imidazolyl,

$Q_1$ Ethylen, Propylen oder 2,2-Dimethylpropylen bedeuten und

$An^{\ominus}$ die angegebene Bedeutung hat.

Dabei sind quaternäre Ammoniumsalze der Formel (2), worin $R_4$ und $R_5$ identisch sind und Methyl oder Ethyl darstellen und $An^{\ominus}$ das Chloridion oder Sulfation ist, bevorzugt.

Die Herstellung der erfindungsgemässen quaternären Ammoniumsalze erfolgt in an sich bekannter Weise. Vorzugsweise kann die Herstellung dadurch erfolgen, dass man das basische Carbamid oder dessen Säuresalz z.B. mit Chlorwasserstoffsäure oder Schwefelsäure, mit einem oe-Epihalogenhydrin, wie z.B. Epibromhydrin, β-Methylepichlorhydrin oder vorzugsweise Epichlorhydrin umsetzt.

Die Bedingungen für die Quaternisierung sind so zu wählen, dass weder infolge zu hoher pH-Werte des Reaktionsmediums noch infolge zu hoher Temperatur ein vorzeitiger Austausch beweglicher Substituenten eintritt. Man arbeitet daher bevorzugt in verdünntem, wässerigem Medium unter möglichst schonender Temperatur und pH-Verhältnissen, zweckmässig bei einer Temperatur von 30 bis 85°C und einem pH-Wert von 6 bis 8,5 vorzugsweise 7 bis 8, wobei zur Erzielung des erwünschten pH-Wertes und zur Bildung des Hydrohalogenidsalzes eine Halogenwasserstoffsäure, vorzugsweise Chlorwasserstoffsäure verwendet wird.

Die zur Quaternisierung benötigten basischen Carbamide können durch Umsetzung einer entsprechenden N-substituierten Diaminoverbindung, z.B. 3-Dimethylaminopropylamin, mit einer Harnstoffverbindung unter Abspaltung von Ammoniak erhalten werden. Die Additionssalze können unter Zugabe einer anorganischen oder organischen Säure zu den freien Aminen erhalten werden.

Als besonders interessante Ausgangsstoffe haben sich basische Carbamide der Formel

$(CH_3)_2N-CH_2CH_2CH_2-NH-CO-NH_2$

$(CH_3)_2N-CH_2CH_2-NH-CO-NH_2$

$(CH_3)_2N-CH_2CH_2CH_2-NH-CO-NH-CH_2O-H$

$(CH_2H_5)_2N-CH_2CH_2-NH-CO-NH_2$

$(CH_2H_5)_2N-CH_2CH_2CH_2-NH-CO-NH_2$

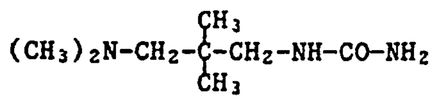

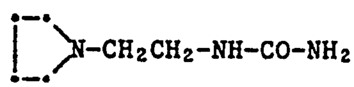

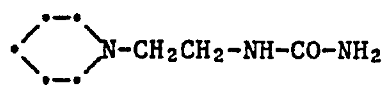

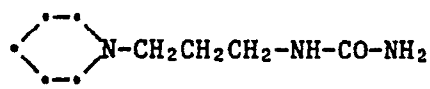

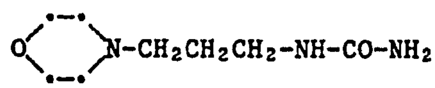 und

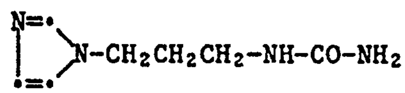

erwiesen.

Erfindungsgemässe quaternäre Ammoniumsalze eignen sich insbesondere zur Verbesserung der Farbausbeute und der Nassechtheiten von Färbungen oder Drucken, welche auf Cellulosefasermaterialien mit anionischen Farbstoffen, wie z.B. Reaktiv- oder Direktfarbstoffen erzeugt werden.

Die Behandlung des Cellulosematerials wird vorzugsweise halbkontinuierlich nach der Kaltverweilmethode durchgeführt. Dabei wird das Cellulosematerial mit dem Behandlungsmittel (Fixiermittel) imprägniert z.B. durch Bedrucken oder vorzugsweise Klotzen und dann durch Lagerung einem Fixierprozess unterworfen. Diese Applikation kann vor dem Färben oder während des Färbens durchgeführt werden. Vorzugsweise erfolgt die Behandlung nach dem Klotz-Kaltverweilverfahren und insbesondere während des Färbens.

Die Imprägnierung kann bei 20 bis 50°C, vorzugsweise jedoch bei Raumtemperatur vorgenommen werden. Der Fixierprozess erfolgt durch Lagern der imprägnierten Ware während 4 bis 48 Stunden, vorzugsweise 10 bis 25 Stunden bei Raumtemperatur.

Die Zubereitungen (Klotzflotten oder Druckpasten) enthalten das quaternäre Ammoniumsalz der Formel (1) vorteilhafterweise in einer Menge von 10 bis 70 g/l, vorzugsweise 25 bis 50 g/l Wirkstoffgehalt. Bei den Klotzflotten beträgt der Abquetscheffekt zweckmässig 60 bis 120 Gew.-%.

Ausser der kationischen, reaktiven Verbindung der Formel (1) enthalten diese Zubereitungen noch alkalisch reagierende Verbindungen, wie z.B. Kaliumhydroxid oder vorzugsweise Natriumhydroxid. Bevorzugt wird eine 30%-ige wässerige Natriumhydroxidlösung, die in einer Menge von 20 bis 50 ml/l, vorzugsweise 25 bis 40 ml/l der Zubereitung zugesetzt wird.

Der pH-Wert der Zubereitungen kann somit in der Regel 8 bis 13,5, vorzugsweise 10 bis 12 betragen.

Die Zubereitungen können auch weitere übliche Zusätze z.B. Elektrolyte, wie z.B. Natriumchlorid oder Natriumsulfat, Harnstoff, Glycerin, Verdicker, wie z.B. Alginate, Stärkeether oder Polyacrylate, Reduktionsschutzmittel, Dispergier- und Netzmittel, Homopolymerisate oder Mischpolymerisate des Acrylamids oder Methacrylamids oder Pfropfpolymerisate, wie sie in EP-A-111 454 beschrieben sind, sowie auch Entschäumer und weitere kationische Fixiermittel enthalten, welch letztere auch faserreaktiv sein können.

Als Fasermaterial kann regenerierte oder insbesondere natürliche Cellulose in Betracht kommen, wie z.B. Zellwolle, Viskoseseide, Hanf, Leinen, Jute oder vorzugsweise Baumwolle, sowie auch Fasermischungen mit synthetischen Fasern z.B. solche aus Polyamid/Baumwolle oder insbesondere aus Polyester/Baumwolle.

Das Textilgut ist in jeglicher Form anwendbar, wie z.B. Garne, Garnstränge, Gewebe, Gewirke, Filze, vorzugsweise in Form von textilen Flächengebilden, wie Gewebe, Maschenware oder Teppich, die ganz oder teilweise aus nativer, regenerierter oder modifizierter Cellulose bestehen.

Die Vorbehandlung des Cellulosefasermaterials mit den erfindungsgemässen kationischen Verbindungen kann mit anderen Vorbehandlungsoperationen gekoppelt werden. Man kann z.B. dem alkalischen Bad, in dem Rohbaumwolle zur Entfernung von Verunreinigungen üblicherweise vor dem Färben abgekocht wird, das erfindungsgemässe reaktive Fixiermittel zusetzen und so die Reinigung und die Vorbehandlung mit dem Fixiermittel in einem Arbeitsgang durchführen.

Vorzugsweise erfolgt die Behandlung des Cellosefasermaterials gleichzeitig mit dem Färben. Die Färbungen erfolgen mit Reaktivfarbstoffen oder vorzugsweise mit Substantivfarbstoffen nach dem Kaltverweilverfahren, wobei die Imprägnierung sowohl durch Bedrucken als auch durch Klotzen durchgeführt werden kann.

Die Menge der Farbstoffe richtet sich in der Regel nach der gewünschten Farbstärke und beträgt zweckmässig 0,1 bis 100 g pro Liter Flotte, vorzugsweise 5 bis 40 g/l Flotte.

Im Falle der Verwendung der kationischen Verbindung in einer Vorbehandlung des Cellulosefasermaterials kann die Färbung nach dem Ausziehverfahren oder durch zweistufige Prozesse, wie z.B. das Foulardierverfahren oder Bedrucken erzeugt werden, wobei als Foulardierverfahren, besonders das sogenannte Pad-Steam-Verfahren, Pad-Fix-Verfahren oder das Klotz-Kaltverweilverfahren in Frage kommen können.

Als Substantivfarbstoffe sind die üblichen Direktfarbstoffe geeignet, beispielsweise die in Colour Index, 3. Auflage (1971) Band 2 auf den Seiten 2005-2478 genannten "Direct Dyes".

Unter Reaktivfarbstoffen werden die üblichen Farbstoffe verstanden, welche mit der Cellulose eine chemische Bindung eingehen, z.B. die in Colour Index, in Band 3 (3. Auflage, 1971) auf den Seiten 3391-3560 und in Band 6 (revidierte 3. Auflage, 1975) auf den Seiten 6268-6345 aufgeführten "Reactive Dyes".

Man erhält nach dem erfindungsgemässen Verfahren sowohl bei der Vorbehandlung mit anschliessendem Färben als auch bei gleichzeitiger Applikation des kationischen Fixiermittels und Farbstoffes gleichmässige und farbkräftige Ausfärbungen, die sich gegenüber bekannten Färbemöglichkeiten durch eine verbesserte Farbausbeute auszeichnen. Insbesondere werden Färbungen auf Cellulosefasermaterial mit Substantivfarbstoffen erzeugt, welche eine erhebliche Verbesserung der Nassechtheiten zeigen.

In den folgenden Herstellungs- und Anwendungsbeispielen beziehen sich die Prozentansätze, wenn nichts anderes angegeben ist, auf das Gewicht. Die Mengen beziehen sich bei den Farbstoffen auf handelsübliche, d.h. coupierte Ware und bei den Hilfsmitteln auf Reinsubstanz. Die fünfstelligen Colour-Index Nummern (C.I) beziehen sich auf die 3. Auflage des Colour-Index.

## Herstellungsbeispiele

### Beispiel 1 :

72,5 g Dimethylaminopropylharnstoff werden in 49 g Wasser gelöst und bei 20°C mit einer Lösung aus 20 g Wasser und 49,3 g 37%iger Salzsäure versetzt. Die Lösung wird auf 60°C erwärmt. Hierauf lässt man in 30 Minuten 46,25 g Epichlorhydrin zutropfen, wobei die Temperatur auf 72°C ansteigt. Nach beendetem Zutropfen wird bei 65°C 5 Stunden nachgerührt.

Man erhält 237 g einer Lösung einer Verbindung der Formel

(11) 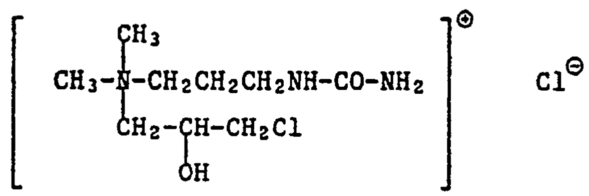

wobei der Epoxyd- und Amingehalt 0 beträgt.

Der pH Wert beträgt 6.1.

Beispiel 2 :

89 g Dimethylaminoneopentylharnstoff werden in einer Lösung aus 86 g Wasser und 49,3 g 37%iger Salzsäure gelöst und auf 60°C erwärmt. Dann lässt man in 30 Minuten 46,25 g Epichlorhydrin zutropfen, wobei die Temperatur auf 65°C ansteigt. Nach beendetem Zufluss wird bei 65°C 5 1/2 Stunden nachgerührt. Nach dieser Zeit betragen Epoxyd- und Amingehalt 0. Man erhält 270 g einer klaren, leicht gelblichen Lösung einer Verbindung der Formel

(12) 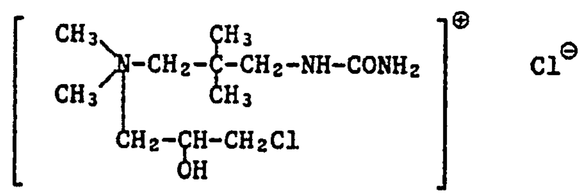

wobei der pH-Wert 6.5 beträgt.

Beispiel 3 :

76,0 g [2-(1-Pyrrolidinyl)ethyl]-harnstoff werden in 55,4 g Wasser gelöst und bei 20°C mit einer Lösung aus 20 g Wasser und 49,3 g 37%iger Salzsäure versetzt. Die Lösung wird auf 60°C erwärmt, worauf man in 30 Minuten 46,25 g Epichlorhydrin zutropfen lässt. Dabei steigt die Temperatur auf 65°C an. Nach beendetem Zufluss wird bei 65°C 4 Stunden nachgerührt.

Nach dieser Zeit betragen Amin- und Epoxydgehalt 0. Man erhält 247 g einer klaren, leicht gelblichen Lösung, einer Verbindung der Formel

(13) 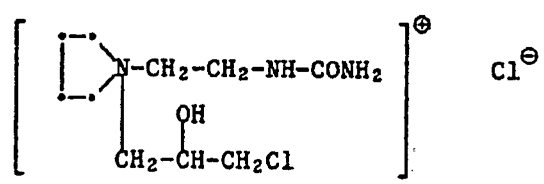

wobei der pH-Wert 6.0 beträgt.

Beispiel 4 :

83 g [3-(1-Imidazolyl)-propyl-]harnstoff werden in 79,9 g Wasser gelöst und bei 20°C mit einer Lösung aus 20 g Wasser und 49,3 g 37%iger Salzsäure versetzt. Danach wird die Lösung auf 60°C erwärmt. Alsdann lässt man in 30 Minuten 46,25 g Epichlorhydrin zutropfen, wobei die Temperatur auf 74°C ansteigt. Nach beendetem Zufluss wird noch 2 Stunden bei 70°C nachgerührt. Nach dieser Zeit beträgt die Aminzahl 22 und der Epoxydgehalt 0. Man erhält 278,5 g einer klaren, gelblichen Lösung einer Verbindung der Formel

(14) 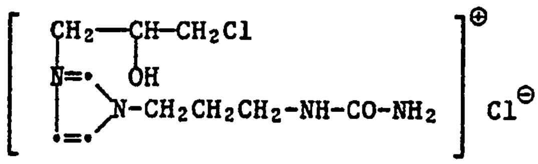

wobei der pH-Wert 6.5 beträgt.

Beispiel 5 :

94,5 g [3-(1-Morpholinyl)-propyl-]harnstoff werden in 94,5 g Wasser gelöst und bei 20°C mit einer Lösung aus 67,3 g Wasser und 49,3 g 37%iger Salzsäure versetzt. Danach wird die Lösung auf 60°C erwärmt. Alsdann lässt man in 30 Minuten 46,25 g Epichlorhydrin zutropfen, wobei die Temperatur auf 64°C ansteigt. Nach beendigtem Zutropfen wird noch 8 Stunden bei 70°C nachgerührt. Nach dieser Zeit betragen Amin- und Epoxydgehalt 0. Man erhält 350 g einer Lösung einer Verbindung der Formel

(15) 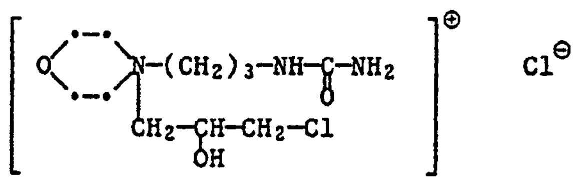

wobei der pH-Wert 7,0 beträgt.

Beispiele 6 :

86,2 g [2-(1-Piperidinyl)-ethyl-]harnstoff werden in 63,1 g Wasser gelöst und bei 20°C mit einer Lösung aus 20 g Wasser und 49,3 g 37%iger Salzsäure versetzt. Danach wird die Lösung auf 60°C erwärmt. Alsdann lässt man in 30 Minuten 46,25 g Epichlorhydrin zutropfen, wobei die Temperatur auf 67°C ansteigt. Nach beendetem Zufluss wird 10 Stunden bei 70°C nachgerührt.

Nach dieser Zeit betragen Amin- und Epoxydgehalt 0. Man erhält 264 g einer Lösung einer Verbindung der Formel

(16) 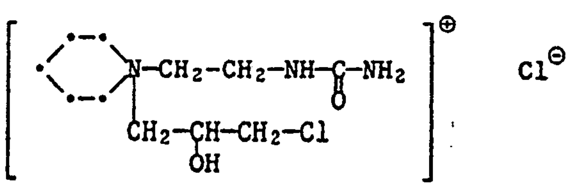

deren pH-Wert 7,5 beträgt.

Beispiel 7 :

91,8 g Diethylaminopropylharnstoff werden in 68,7 g Wasser gelöst und bei 20°C mit einer Lösung aus 20 g Wasser und 49,3 g 37%iger Salzsäure versetzt. Die Lösung wird auf 60°C erwärmt. In 30 Minuten lässt man 46,25 g Epichlorhydrin zutropfen, wobei die Temperatur auf 65°C ansteigt. Nach beendetem Zutropfen wird bei 65°C 5 Stunden nach gerührt. Nach dieser Zeit betragen Amin- und Epoxydgehalt 0. Man erhält 275 g einer Lösung einer Verbindung folgender Formel

(17)

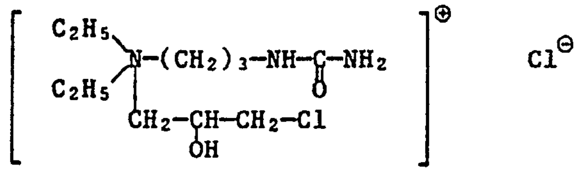

Der pH-Wert beträgt 7,6.

Beispiel 8 :

70,5 g Dimethylaminopropylharnstoff werden in 71 g Wasser gelöst und bei 10°C mit einer Lösung aus 20 g Wasser und 25,5 g 96%ige Schwefelsäure versetzt. Die Lösung wird auf 70°C erwärmt. Hierauf lässt man in 30 Minuten 46,25 g Epichlorhydrin zufliessen wobei die Temperatur auf 80°C ansteigt. Nach beendetem Zutropfen wird 10 Stunden bei 80°C nachgerührt. Nach dieser Zeit betragen Amin- und Epoxydgehalt 0. Man erhält 232 g einer Lösung einer Verbindung folgender Formel

(18)

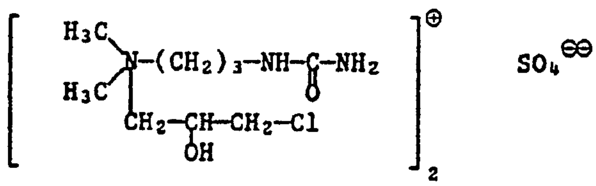

Der pH-Wert beträgt 7,2.

Beispiel 9 :

77 g N-(3-Dimethylaminopropyl)-N'-methylol-harnstoff werden in 54,3 g Wasser gelöst und bei 10°C mit einer Lösung aus 20 g Wasser und 43,4 g 37%igen Salzsäure versetzt. Die Lösung wird auf 45°C erwärmt. Hierauf lässt man in 30 Minuten 40,7 g Epichlorhydrin zutropfen, wobei die Temperatur auf 50°C ansteigt. Nach beendetem Zutropfen wird 5 Stunden bei 50°C nachgerührt. Nach dieser Zeit betragen Amin- uund Epoxydgehalt 0. Man erhält 235 g einer Lösung einer Verbindung der Formel

(19)

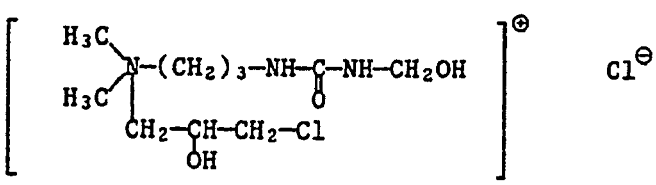

Der pH-Wert beträgt 7,0.

Anwendungsbeispiele

Beispiel 1 :

Jeweils 20 g Baumwoll-Cretonne, gebleicht und nicht mercerisiert werden getrennt auf einem Foulard mit einer der 4 folgenden Flotten, welche im Liter

1) 20 g des Farbstoffes Direct Red 80 C.I. 35780
32 ml Natriumhydroxidlösung (30%)
35 g des quaternären Ammoniumsalzes der Formel (11)
2) 12 g des Farbstoffes Direct Blue 71 C.I. 34140
32 ml Natriumhydroxidlösung (30%)
35 g des quaternären Ammoniumsalzes der Formel (11)
3) 12 g des Farbstoffes Direct Violet 66 C.I. 29125
32 ml Natriumhydroxidlösung (30%)
35 g des quaternären Ammoniumsalzes der Formel (11)

4) 20 g des Farbstoffes Direct Green 26 C.I. 34045
32 ml Natriumhydroxidlösung (30%)
35 g des quaternären Ammoniumsalzes der Formel (11)
enthalten, imprägniert. Die Flottenaufnahme beträgt jeweils 80%. Hiernach werden die Gewebe nass aufgerollt und luftdicht verpackt 18 Stunden bei Raumtemperatur gelagert. Anschliessend wird die Ware kalt und heiss gespült und getrocknet.
Von diesen 4 Färbungen werden folgende Echtheiten geprüft :

- Nassbügelechtheit (SN ISO 105-X11)

- ISO C2S-Wäsche (ISO 105-CO6)

wobei auch entsprechende stärkegleiche Färbungen, die jeweils ohne Zusatz des quaternären Ammoniumsalzes der Formel (11) erhalten worden sind, mitgeprüft werden.
In der folgenden Tabelle 1 sind die Echtheitsbewertungen aufgeführt.

Tabelle 1

| Färbungen | g/l Farbstoff | Nassbügel-echtheit | ISO C2S-Wäsche | |
|---|---|---|---|---|
| | | | Aendern der Nuance | Bluten auf Baumwolle |
| (1) ohne | 45 | 3-4 | 4 | 2 |
| mit | 20 | 5 | 5 | 3-4 |
| (2) ohne | 16 | 2 | 4 | 2 |
| mit | 12 | 5 | 5 | 5 |
| (3) ohne | 25 | 2-3 | 4-5 | 4 |
| mit | 12 | 4-5 | 5 | 5 |
| (4) ohne | 35 | 4-5 | 4-5 | 3-4 |
| mit | 20 | 5 | 5 | 5 |

Beispiel 2 :

Jeweils 20 g Baumwoll-Tricot, gebleicht und mercerisiert, werden getrennt auf einem Foulard mit einer der 4 folgenden Flotten, welche im Liter
1) 25 g eines Farbstoffes der Formel

(101)

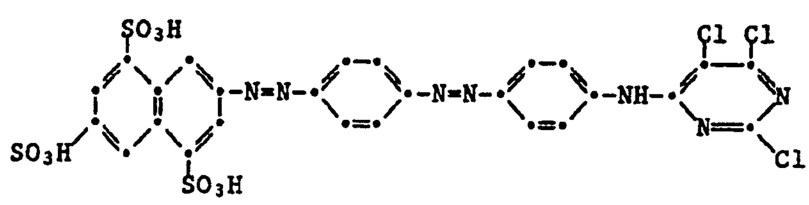

50 g des quaternären Ammoniumsalzes der Formel (11)
40 ml Natriumhydroxidlösung (30%)
100 g Harnstoff und
3 g Natriumsalz der 3-Nitrobenzolsulfonsäure
2) 25 g eines Farbstoffes der Formel

(102) 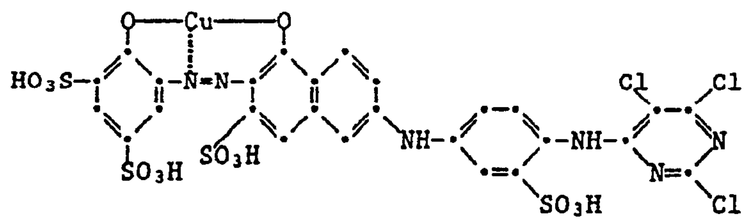

50 g des quaternären Ammoniumsalzes der Formel (11)
40 ml Natriumhydroxidlösung (30%)
100 g Harnstoff und
3 g Natriumsalz der 3-Nitrobenzolsulfonsäure
3) 25 g eines Farbstoffes der Formel

(103) 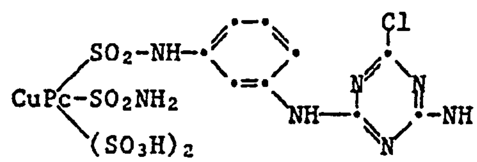

50 g des quaternären Ammoniumsalzes der Formel (11)
40 ml Natriumhydroxidlösung (30%)
100 g Harnstoff und
3 g Natriumsalz der 3-Nitrobenzolsulfonsäure
4) 25 g eines Farbstoffes der Formel

(104) 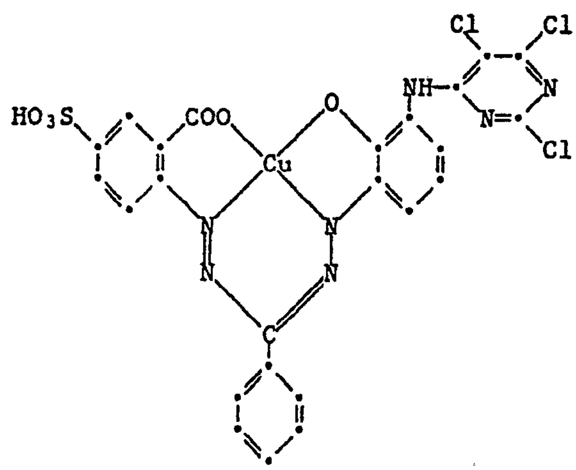

50 g des quaternären Ammoniumsalzes der Formel (11)
40 ml Natriumhydroxidlösung (30%)
100 g Harnstoff und
3 g Natriumsalz der 3-Nitrobenzolsulfonsäure enthalten, imprägniert. Die Flottenaufnahme beträgt jeweils 100%. Hierauf werden die Tricotstücke nass aufgerollt und luftdicht verpackt 18 Stunden gelagert.

Anschliessend wird die Ware kalt und heiss gespült und getrocknet.

Von diesen 4 Färbungen werden die Nassbügelechtheit und die ISO C2S-Wäsche geprüft, wobei auch entsprechende stärkegleiche, jeweils mit mehr Farbstoff erhaltenen Färbungen, die jeweils ohne Zusatz des quaternären Ammoniumsalzes der Formel (11) erhalten worden sind, mitgeprüft werden.

In der folgenden Tabelle 2 sind die Echtheitsbewertungen gegenübergestellt.

Tabelle 2

| Färbungen | g/l Farbstoff | Nassbügel-echtheit | ISO C2S-Wäsche | |
|---|---|---|---|---|
| | | | Aendern der Nuance | Bluten auf Baumwolle |
| (1) ohne | 40 | 4-5 | 5 | 4-5 |
| mit | 25 | 5 | 5 | 4-5 |
| (2) ohne | 37 | 4 | 5 | 4-5 |
| mit | 25 | 5 | 5 | 4-5 |
| (3) ohne | 35 | 4-5 | 5 | 4 |
| mit | 25 | 5 | 5 | 5 |
| (4) ohne | 38 | 4-5 | 5 | 5 |
| mit | 25 | 5 | 5 | 5 |

Aehnlich gute Effekte erhält man bei Verwendung von 50 g/l jedes der gemäss den Beispielen 2 bis 4 hergestellten quaternären Ammoniumsalzes.

Beispiel 3 :

Je 20 g Baumwolltricot, gebleicht und mercerisiert, werden bei einer Flottenaufnahme von 90% mit einer Zubereitung foulardiert, die im Liter
35 g des quaternären Ammoniumsalzes der Formel (11)
30 ml Natriumhydroxidlösung (30%)
enthält. Nach dem Foulardieren wird das Tricot nass aufgerollt und in einem Plastiksack 18 Stunden bei Raumtemperatur gelagert. Anschliessend wird die Ware kalt und heiss gespült.

Das vorbehandelte Tricot wird zusammen mit 20 g nicht-behandeltem Tricot in einer wässerigen Färbeflotte bei 50°C eingenetzt, welche bei einem Flottenverhältnis von 1 : 40 1% des Farbstoffes Direct Blue 71 C.I. 43140 enthält. Man steigert innert 30 Minuten die Temperatur auf 98°C und färbt 45 Minuten bei dieser Temperatur.

Man erhält 2 Tricotstücke, wobei das vorbehandelte Stück tiefblau gefärbt ist, während das nicht-vorbehandelte Material nur leicht angefärbt ist.

Beispiel 4 :

Das gemäss Beispiel 3 vorbehandelte Tricot wird zusammen mit 20 g nicht-vorbehandeltem Tricot bei 98°C in einer wässerigen Flotte eingenetzt, welche bei einem Flottenverhältnis von 1 : 30
1% des Farbstoffes der Formel

(105) 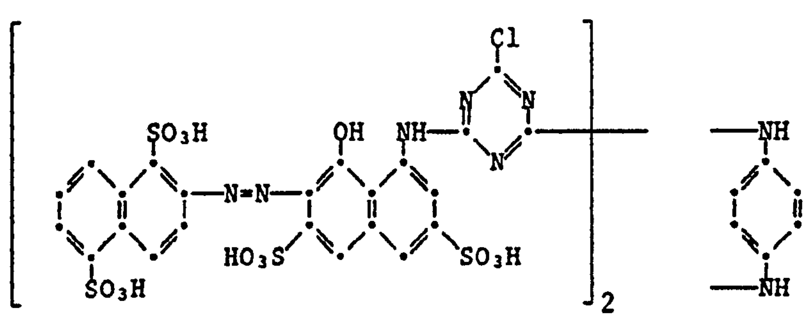

enthält. Hierauf senkt man die Temperatur innert 30 Minuten auf 85°C, fügt
5 g/l Natriumcarbonat calc.
2 ml/l Natriumhydroxidlösung (30%)

hinzu und behandelt das Material weitere 45 Minuten bei 85°C. Anschliessend werden die Färbungen 5 Minuten in kochendem Wasser gespült.

Man erhält 2 Tricots, wobei das vorbehandelte Tricot tiefrot gefärbt ist, während das nichtvorbehandelte Material nur leicht rosa gefärbt ist.

Beispiel 5 :

Das gemäss Beispiel 3 vorbehandelte Tricot wird zusammen mit 20 g nicht-vorbehandeltem Tricot und 20 g von auf gleiche Weise, jedoch nur mit 30 ml/l Natriumhydroxidlösung (30%) behandeltem Tricot in einer wässerigen Flotte bei 50°C eingenetzt, die bei einem Flottenverhältnis von 1 : 40, 1% eines Farbstoffes der Formel

(106) 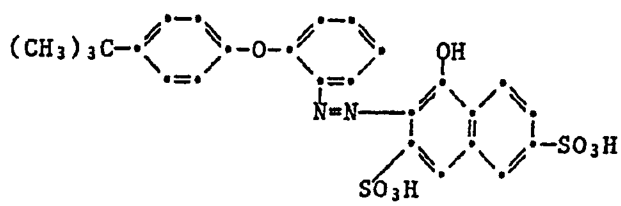

enthält. Hierauf färbt man das Material 40 Minuten bei 50°C und spült es anschliessend 5 Minuten warm.

Man erhält 3 Tricotstücke, wobei das gemäss Beispiel 3 vorbehandelte Tricot tiefrot gefärbt ist, während die 2 übrigen Tricotstücke nur leicht angefärbt sind.

Beispiel 6 :

Jeweils 20 g Baumwolltricot, gebleicht und mercerisiert, werden getrennt auf einem Foulard mit einer der 4 folgenden Flotten, welche im Liter

1) 18 g des Farbstoffes der Formel

(107) 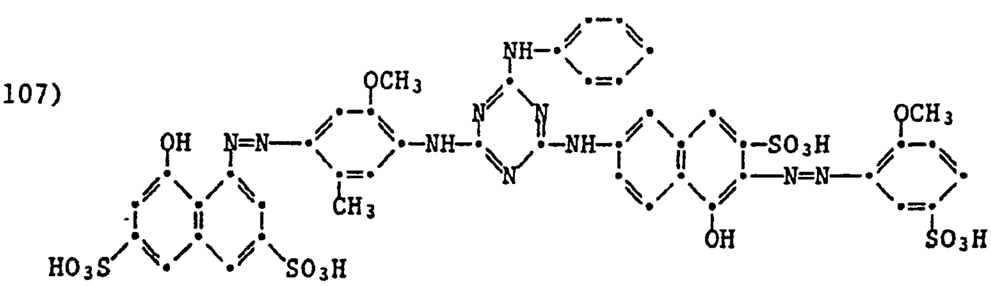

32 ml Natriumhydroxidlösung 30%
35 g des quaternären Ammoniumsalzes der Formel (19)
2) 18 g des Farbstoffes Direct Red 80 C.I. 35780
32 ml Natriumhydroxidlösung 30%
35 g des quaternären Ammoniumsalzes der Formel (19)
3) 8 g des Farbstoffes Direct Blue 71 C.I. 34140
32 ml Natriumhydroxidlösung 30%
35 g des quaternären Ammoniumslazes der Formel (19)
4) 8 g des Farbstoffes der Formel

(108) 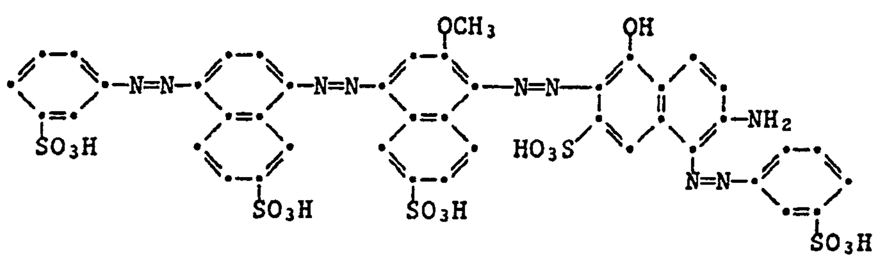

32 ml Natriumhydroxidlösung 30%
35 g des quaternären Ammoniumsalzes der Formel (19) enthält, imprägniert. Die Flottenaufnahme beträgt jeweils 90%. Hiernach werden die Tricots nass aufgerollt, luftdicht verpackt und 18 Stunden bei Raumtemperatur gelagert. Dann wird die Ware kalt und heiss gespült und getrocknet.

<u>Beispiel 7 :</u>

Die 4 Färbungen von Anwendungsbeispiel 6 werden wiederholt wobei jedoch an Stelle des quaternären Ammoniumsalzes der Formel (19)
jeweils 1. 35 g/l des quaternären Ammoniumsalzes der Formel (18)
2. 35 g/l des quaternären Ammoniumsalzes der Formel (15)
3. 35 g/l des quaternären Ammoniumsalzes der Formel (16)
a4. 35 g/l des quaternären Ammoniumsalzes der Formel (17)
eingesetzt werden.

Von den 20 Färbungen aus den Anwendungsbeispielen 6 und 7 werden folgende Echtheiten geprüft :

- Nassbügelechtheit
- ISO C2S-Wäsche

wobei auch entsprechende stärkegleiche Färbungen, die jeweils ohne Zusatz der quaternären Ammoniumsalze erhalten worden sind, mitgeprüft werden.
In der folgenden Tabelle 3 sind die Echtheitswerte aufgeführt.

Tabelle 3

| Färbungen | g/l Farbstoff für stärkegleiche Färbungen | Nassbügelechtheit | ISO C2S-Wäsche | |
|---|---|---|---|---|
| | | | Aendern der Nuancen | Bluten auf Baumwolle |
| (1) ohne | 25 | 3 | 4 | 2-3 |
| Formel (19) | 18 | 5 | 4-5 | 4 |
| Formel (18) | 18 | 5 | 4-5 | 4 |
| Formel (15) | 18 | 5 | 4-5 | 3-4 |
| Formel (16) | 18 | 5 | 5 | 4 |
| Formel (17) | 18 | 5 | 4-5 | 4 |
| (2) ohne | 38 | 3 | 4 | 2 |
| Formel (19) | 18 | 5 | 4-5 | 4 |
| Formel (18) | 18 | 5 | 4-5 | 4 |
| Formel (15) | 18 | 5 | 4-5 | 3-4 |
| Formel (16) | 18 | 5 | 4-5 | 3-4 |
| Formel (17) | 18 | 5 | 5 | 4 |
| (3) ohne | 14 | 2 | 4 | 2 |
| Formel (19) | 8 | 5 | 4-5 | 4-5 |
| Formel (18) | 8 | 5 | 4-5 | 4-5 |
| Formel (15) | 8 | 5 | 4-5 | 4 |
| Formel (16) | 8 | 5 | 4-5 | 3-4 |
| Formel (17) | 8 | 5 | 5 | 4 |
| (4) ohne | 16 | 3 | 4-5 | 3-4 |
| Formel (19) | 8 | 5 | 5 | 5 |
| Formel (18) | 8 | 5 | 5 | 5 |
| Formel (15) | 8 | 5 | 5 | 4-5 |
| Formel (16) | 8 | 5 | 5 | 5 |
| Formel (17) | 8 | 5 | 5 | 4-5 |

**Ansprüche**

1. Quaternäre Ammoniumsalze, welche der Formel

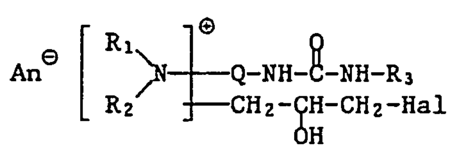

(1)

entsprechen,

worin Q Alkylen mit 2 bis 6 Kohlenstoffatomen,

$R_1$ und $R_2$, unabhängig voneinander, je unsubstituiertes oder durch Halogen, Hydroxyl, Cyano oder Niederalkoxy substituiertes Niederarkyl, Cycloalkyl oder Aralkyl,

oder $R_1$ und $R_2$ zusammen mit dem sie verbindenden Stickstoffatom einen fünf- oder sechsgliedrigen heterocyclischen Rest, der gegebenenfalls ein weiteres Heteroatom enthält,

$R_3$ Wasserstoff, Niederarkyl oder Hydroxyniederalkyl,

Hal ein Halogenatom,

und $An^{\ominus}$ das Anion einer starken anorganischen oder organischen Säure bedeuten und die Halogenhydringruppe an ein quaternisiertes Stickstoffatom gebunden ist, wobei der Ausdruck "Nieder" sich auf solche Gruppen mit 1 bis 5 Kohlenstoffatomen bezieht.

2. Ammoniumsalze gemäss Anspruch 1 dadurch gekennzeichnet, dass in Formel (1) Q die Ethylen-, Propylen- oder 2,2-Dimethylpropylengruppe bedeutet.

3. Ammoniumsalze gemäss einem der Ansprüche 1 und 2 dadurch gekennzeichnet, dass in Formel (1) $R_3$ Wasserstoff bedeutet.

4. Ammoniumsalze gemäss Anspruch 1 dadurch gekennzeichnet, dass sie der Formel

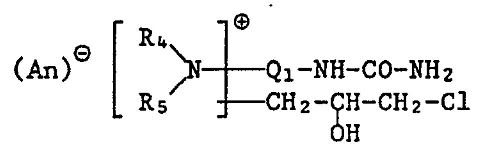 (2)

entsprechen, worin

$R_4$ und $R_5$ unabhängig voneinander, je Niedetarkyl, besonders Methyl oder Ethyl, oder $-NR_4R_5$ Pyrrolidino, Piperidino, Morpholino oder Imidazolyl,

$Q_1$ Ethylen, Propylen oder 2,2-Dimethylpropylen und

$An^{\ominus}$ das Anion einer starken anorganischen oder organischen Säure bedeuten.

5. Ammoniumsalz gemäss Anspruch 1 dadurch gekennzeichnet, dass es der Formel

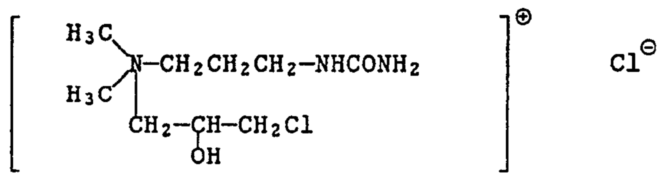

entspricht

6. Ammoniumsalz gemäss Anspruch 1 dadurch gekennzeichnet, dass es der Formel

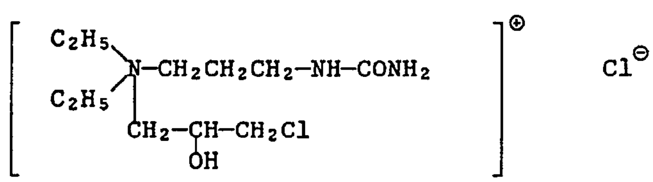

entspricht

7. Ammoniumsalz gemäss Anspruch 1 dadurch gekennzeichnet, dass es der Formel

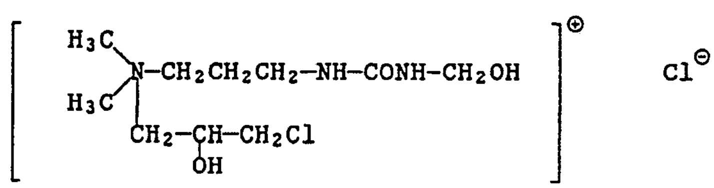

entspricht

8. Verwendung der quaternären Ammoniumsalze gemäss einem der Ansprüche 1 bis 7 zur Verbesserung der Farbausbeute und der Nassechtheiten von mit anionischen Farbstoffen auf Cellulosefasermaterialien erzeugten Färbungen oder Drucken.

9. Verfahren zur Verbesserung der Farbausbeute und der Nassechtheiten von mit anionischen Farbstoffen auf Cellulosefasermaterial erzeugten Färbungen oder Drucken, dadurch gekennzeichnet, dass man das Fasermaterial vor dem Färben oder während des Färbens mit einem quaternären Ammoniumsalz gemäss einem der Anspsüche 1 bis 7 behandelt.

10. Verfahren gemäss Anspruch 9, dadurch gekennzeichnet, dass die Behandlung halbkontinuierlich

gemäss dem Kaltverweilverfahren durchgeführt wird.

11. Verfahren gemäss einem der Ansprüche 9 und 10 dadurch gekennzeichnet, dass die Behandlung gemäss dem Klotz-Kaltverweilverfahren und während des Färbens durchgeführt wird.

12. Verfahren gemäss einem der Ansprüche 9 bis 11 dadurch gekennzeichnet, dass die Behandlung aus alkalischem Medium erfolgt.

13. Verfahren gemäss einem der Ansprüche 9 bis 12 dadurch gekennzeichnet, dass die Behandlung zur Verbessetung der Farbausbeute und der Nassechtheiten von mit substantiven Farbstoffen erzeugten Färbungen durchgeführt wird.

## Revendications

1. Sels d'ammonium quaternaire qui correspondent à la formule

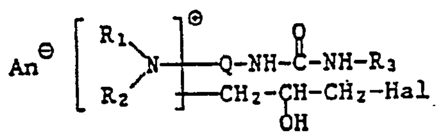

(1)

dans laquelle

Q représente un groupe alkylène ayant de 2 à 6 atomes de carbone ;

$R_1$ et $R_2$ représentent, indépendamment l'un de l'autre, un radical alkyle inférieur, cycloalkyle ou aralkyle, chacun non substitué ou substitué par un atome d'halogène ou par un groupe hydroxy, cyano ou alcoxy, inférieur ;

ou $R_1$ et $R_2$ représentent ensemble, avec l'atome d'azote auquel ils sont liés, un radical hétérocyclique à 5 ou 6 chaînons, qui contient éventuellement un autre hétéroatome ;

$R_3$ représente un atome d'hydrogène ou un groupe alkyle inférieur ou hydroxy-alkyle inférieur ;

Hal représente un atome d'halogène ; et

$An^{\ominus}$ représente l'anion d'un acide fort organique ou minéral ; et le groupe halohydrine est lié à un atome d'azote quaternaire, l'expression "inférieur" se rapportant à de tels groupes ayant de 1 à 5 atomes de carbone.

2. Sels d'ammonium selon la revendication 1, caractérisés en ce que, dans la formule (1), Q représente le groupe éthylène, propylène ou 2,2-diméthylpropylène.

3. Sels d'ammonium selon l'une des revendications 1 et 2, caractérisés en ce que, dans la formule (1), $R_3$ représente un atome d'hydrogène.

4. Sels d'ammonium selon la revendication 1, caractérisés en ce qu'ils corespondent à la formule

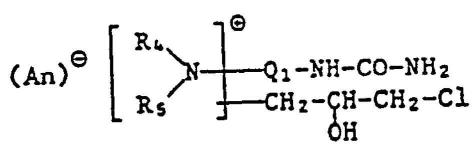

(2)

dans laquelle

$R_4$ et $R_5$ représentent chacun, indépendamment l'un de l'autre, un groupe alkyle inférieur, en particulier méthyle ou éthyle, ou $-NR_4R_5$ représente le groupe pyrrolidino, pipéridino, morpholino ou imidazolyle;

$Q_1$ représente le groupe éthylène, propylène ou 2,2-diméthylpropylène ; et

$An^{\ominus}$ représente l'anion d'un acide fort organique ou minéral.

5. Sel d'ammonium selon la revendication 1, caractérisé en ce qu'il correspond à la formule

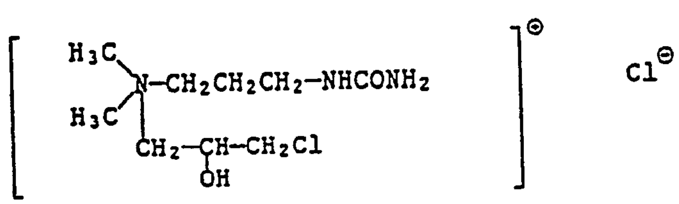

6. Sel d'ammonium selon la revendication 1, caractérisé en ce qu'il correspond à la formule

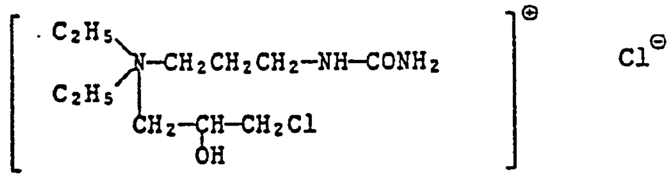

7. Sel d'ammonium selon la revendication 1, caractérisé en ce qu'il correspond à la formule

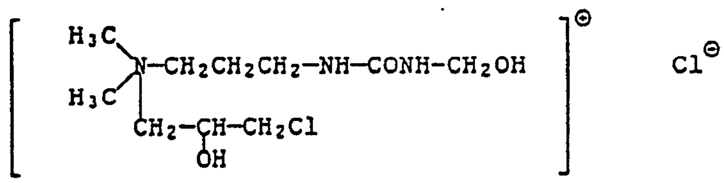

8. Utilisation des sels d'ammonium quaternaire selon l'une des revendications 1 à 7, pour l'amélioration du rendement tinctorial et des solidités au mouillé de teintures ou impressions produites avec des colorants anioniques sur des matériaux fibreux cellulosiques.

9. Procédé pour l'amélioration du rendement tinctorial et des solidités au mouillé de teintures ou impressions produites avec des colorants anioniques sur matériau fibreux cellulosique, caractérisé en ce que, avant ou pendant la teinture, on traite le matériau fibreux par un sel d'ammonium quaternaire selon l'une des revendications 1 à 7.

10. Procédé selon la revendication 9, caractérisé en ce que le traitement est effectué en mode semi-continu selon le procédé d'abandon à froid.

11. Procédé selon l'une des revendications 9 et 10, caractérisé en ce que le traitement est effectué selon le procédé de foulardage-abandon à froid et pendant la teinture.

12. Procédé selon l'une des revendications 9 à 11, caractérisé en ce que le traitement est effectué en milieu alcalin.

13. Procédé selon l'une des revendications 9 à 12, caractérisé en ce que le traitement est effectué pour l'amélioration du rendement tinctorial et des solidités au mouillé de teintures produites avec des colorants substantifs.

**Claims**

1. A quatenary ammonium salt of the formula

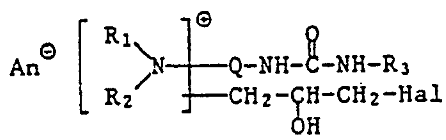

(1)

in which

Q is alkylene of 2 to 6 carbon atoms,

$R_1$ and $R_2$ are each independently of the other, unsubstituted or halogen-, hydroxyl-, cyano- or lower alkoxy-substituted lower alkyl, cycloalkyl or aralkyl,

or $R_1$ and $R_2$ together with the nitrogen atom to which they are attached are a five- or six-membered heterocyclic radical, which optionally contains an additional hetero/atom

$R_3$ is hydrogen, lower alkyl or hydroxy-lower alkyl,

Hal is a halogen atom

and $An^{\ominus}$ is the anion of a strong inorganic or organic acid

and the halohydrin group is bound to a quaternised nitrogen atom, the expression "lower" referring to those groups having 1 to 5 carbon atoms

2. An ammonium salt according to claim 1, wherein in formula (1) Q is the ethylene, propylene or 2,2-dimethylpropylene group.

3. An ammonium salt according to either of claims 1 and 2, wherein in formula (1) $R_3$ is hydrogen.

4. An ammonium salt according to claim 1, of the formula

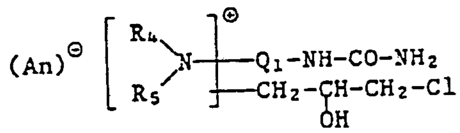

(2)

in which

$R_4$ and $R_5$ are each independently of the other, lower alkyl, particularly methyl or ethyl, or $-NR_4R_5$ pyrrolidino, piperidino, morpholino or imidazolyl,

$Q_1$ is ethylene, propylene or 2,2-dimethylpropylene, and

$An^{\ominus}$ is the anion of a strong inorganic or organic acid.

5. An ammonium salt according to claim 1, of the formula

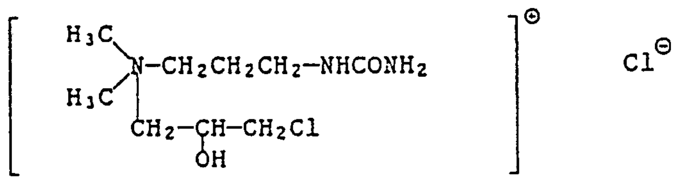

6. An ammonium salt according to claim 1, of the formula

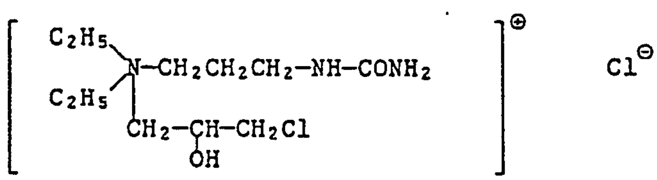

7. An ammonium salt according to claim 1, of the formula

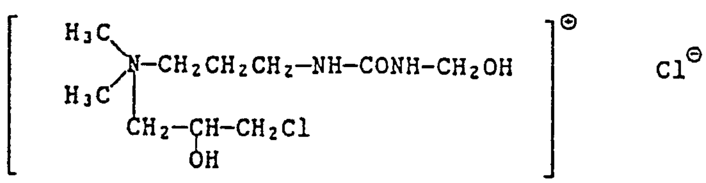

8. Use of quaternary ammonium salts according to any one of claims 1 to 7 for enhancing the colour yield and wet-fastness of dyeings or printings produced with anionic dyes on cellulose fibre materials.

9. A process for enhancing the colour yield and the wet-fastness of dyeings or printings produced with anionic dyes on cellulose fibre material, which comprises treating the fibre material before dyeing or during dyeing with a quaternary ammonium salt according to any one of claims 1 to 7.

10. A process according to claim 9, wherein the treatment is carried out semicontinuously by the cold batch method.

11. A process according to either of claims 9 and 10, wherein the treatment is carried out by the cold pad-batch method and during dyeing.

12. A process according to any one of claims 9 to 11, wherein the treatment is carried out in alkaline medium.

13. A process according to any one of claims 9 to 12, wherein the treatment is carried out for enhancing the colour yield and wet-fastness of dyeings produced with substantive dyes.